Europäisches Patentamt

European Patent Office

Office européen des brevets

(19)

(11) Veröffentlichungsnummer: **0 551 355 B1**

(12) **EUROPÄISCHE PATENTSCHRIFT**

(45) Veröffentlichungstag der Patentschrift: **14.09.94**

(51) Int. Cl.5: **A61N 1/365**

(21) Anmeldenummer: **91917484.7**

(22) Anmeldetag: **04.10.91**

(86) Internationale Anmeldenummer:
**PCT/EP91/01897**

(87) Internationale Veröffentlichungsnummer:
**WO 92/05836 (16.04.92 92/09)**

(54) **ANORDNUNG, INSBESONDERE HERZSCHRITTMACHER, ZUR ERFASSUNG EINES MESSPARAMETERS DER HERZAKTIVITÄT.**

(30) Priorität: **04.10.90 DE 4031450**

(43) Veröffentlichungstag der Anmeldung:
**21.07.93 Patentblatt 93/29**

(45) Bekanntmachung des Hinweises auf die Patenterteilung:
**14.09.94 Patentblatt 94/37**

(84) Benannte Vertragsstaaten:
**DE FR GB IT NL SE**

(56) Entgegenhaltungen:
**EP-A- 0 255 899**
**EP-A- 0 317 985**

(73) Patentinhaber: **Siemens Elema AB**
**Röntgenvägen 2**
**S-171 95 Solna 1 (SE)**

(84) Benannte Vertragsstaaten:
**SE**

(73) Patentinhaber: **SIEMENS AKTIENGESELL-SCHAFT**
**Wittelsbacherplatz 2**
**D-80333 München (DE)**

(84) Benannte Vertragsstaaten:
**DE FR GB IT NL**

(72) Erfinder: **HEINZE, Roland**
**Simbacher Str. 5**
**D-8000 München 80 (DE)**

Rank Xerox (UK) Business Services
(3. 10/3.09/3.3.3)

# Beschreibung

Die Erfindung betrifft eine Anordnung, insbesondere Herzschrittmacher, mit einer Meßeinrichtung zur Erfassung eines Meßparameters der Herzaktivität.

In der internationalen Patentanmeldung WO 89/06990 wird beschrieben, wie es mit Hilfe periodischer Wechsel der Stimulationsfrequenz, der gleichzeitigen Erfassung eines dem Herzminutenvolumen proportionalen Meßparameters und entsprechenden Verrechnungsverfahren möglich ist, eine hämodynamische Optimierung der Frequenzregelung durchzuführen. Wesentlich bei dieser Meßmethode ist aber, daß die Frequenzänderungen über Zeitabstände erfolgen, die nicht nur eine Reaktion des Herzmuskels sondern eine Reaktion des ganzen kardiovaskulären Regelsystems zur Folge hat und daß diese Reaktion Voraussetzung für die angestrebte Optimierung des Frequenzbereiches der Stimulationsfrequenz ist.

Bei einem aus der EP-A-0 255 899 bekannten Herzschrittmacher wird die Stimulationsfrequenz in Abhängigkeit von der atrioventrikulären Überleitungszeit als belastungsabhängiger Führungsgröße geregelt. Dabei ist außer einer Kennlinienregelung eine hämodynamische Optimalregelung vorgesehen, indem bei gleichbleibendem körperlichen Belastungsgrad die Stimulationsfrequenz solange erhöht oder verringert wird, bis die gemessene atrioventrikuläre Überleitungszeit einen Minimalwert aufweist, wobei die so gefundene Stimulationsfrequenz als optimal für den jeweiligen Belastungsgrad angesehen wird. Auch bei diesem Verfahren ist wesentlich, daß die Frequenzänderungen bei der Suche der optimalen Stimulationsfrequenz nicht nur eine Reaktion des Herzmuskels, sondern des ganzen kardiovaskulären Regelsystems zur Folge haben, wobei diese Reaktion Voraussetzung für die angestrebte Optimierung der Stimulationsfrequenz ist.

In der EP-A-0 140 472 wird die Regelung der Stimulationsfrequenz abhängig von Änderungen des Schlagvolumens des Herzens vorgeschlagen, wobei diese Änderungen mit Hilfe der Impedanzmessung bestimmt werden. Die Veröffentlichung enthält jedoch keinen Hinweis darauf, wie mit Hilfe der aus dem Impedanzsignal gewonnenen Meßwerte das Schlagvolumen quantifiziert werden kann. Außerdem ist nicht angegeben, wie die individuellen Unterschiede der Proportionalität zwischen dem Wert des gemessenen Impedanzsignals und dem Grad der Belastung kompensiert werden können.

In der DE-A-35 33 597 ist ein Verfahren beschrieben, bei dem die Stimulationsfrequenz mit einer dem Schlagvolumen proportionalen Meßgröße geregelt wird. Die Regelkennlinie der Stimulationsfrequenz als Funktion des Schlagvolumens wird dabei selbständig dadurch ermittelt, daß bei maximaler Belastung die Frequenz so eingestellt wird, daß das Produkt aus Frequenz und Schlagvolumen ein Maximum wird. Nachteil dieser Methode ist, daß der maximale Belastungszustand durch einen zweiten Meßparameter detektiert oder extern eingegeben werden muß und daß auch kein Hinweis gegeben wird, wie die Störanfälligkeit des Impedanzsignals kompensiert wird, so daß das derartige Verfahren auch keine praktische Anwendung gefunden hat.

Der Erfindung liegt die Aufgabe zugrunde, vorrangig in Verbindung mit Herzschrittmachern, Meßparameter der Herzaktivität so zu erfassen, daß sie von Störsignalen befreit werden, und damit zur Bewertung der physiologischen Funktionen des Herzmuskels insbesondere zur Bestimmung von Änderungen des Herzzeitvolumens genutzt werden können.

Die Lösung dieser Aufgabe erfolgt entsprechend den im Anspruch 1 angegebenen Merkmalen. Bevorzugte Ausführungsbeispiele sind in den Ansprüchen 2-10 definiert.

Im Gegensatz zu den bisherigen in der Schrittmachertechnik benutzten passiven Entstörmethoden wie Frequenzfilterung wird hierzu eine Methode der aktiven Störaustastung angewandt, um den Einfluß von Drift, Sensitivitätsänderung, Linearitätsänderung und Mehrfachsensitivität zu unterdrükken. Dabei handelt es sich im Prinzip darum, durch gezielte Modulation nur des zu untersuchenden Phänomens, etwa nach zeitlichen Kriterien, bei der anschließenden Demodulation die nicht spezifischen Signalanteile herauszufiltern. Das Schrittmacherprinzip bietet dazu die besten Voraussetzungen, da alle Meßparameter der Herzaktivität von der Frequenz des Herzens abhängen und so mit dem Stimulationspuls gezielt modulierbar sind.

Das erfindungsgemäße Konzept nutzt diese Tatsache in der Weise, daß der Signalverlauf eines Meßparameters M während eines Herzpulses $n+1$ abhängig von der Frequenz f bzw. Dauer $t_s$ des vorhergehenden Herzpulses n analysiert wird und daß bei Änderung von f um $\Delta$ f bzw. von $t_s$ um $\Delta t_s$ die Meßwertänderung $\Delta$ M zwischen den Meßwerten $M(n)$ und $M(n+1)$ abhängig vom Grad der Frequenzänderung $\Delta$ f bzw. $\Delta t_s$ zwischen den zwei Pulsen bewertet wird, wobei im Stimulationsfall Meßwertänderungen $\Delta$ M, die durch gezielte Änderungen der Stimulationsfrequenz f hervorgerufen wurden, analysiert werden.

Ist prinzipiell die Abhängigkeit eines Meßparameters M von dem Grad der Frequenzänderung $\Delta$ f physiologisch genau definiert, können alle nicht vom Frequenzwechsel $\Delta$ f abhängigen störenden Signalanteile mit Hilfe frequenzwechselbezogener Auswertung nach den bekannten, einfachen Ausewerteverfahren wie Differenz-, Quotienten- und Mittelwertbildung eliminiert werden.

Damit kann z.B. der physiologische Tatbestand ausgenutzt werden, daß Änderungen der Pulsfrequenz etwa durch Verkürzung oder Verlängerung des Pulsabstandes $t_s$ zwischen zwei Stimulationspulsen eine Beeinflussung der diastolischen Füllungsphase dieses Herzpulses n und dadurch des Verlaufs der systolischen Austreibungsphase während des nächsten Pulses n + 1 bewirken.

Wendet man das Prinzip der frequenzwechselbezogenen Signalauswertung z.B. bei der Analyse von intrakardialen Impedanzmessungen an, deren Nutzung bisher wegen der hohen Störanfälligkeit der Meßmethode mißlang, gelingt es, Veränderungen des Schlagvolumens so störungsfrei zu bewerten, daß mit Hilfe der ausgewerteten Signale u.a. die Stimulationsfrequenz belastungsabhängig geregelt werden kann und/oder die Stimulationsfrequenz hämodynamisch optimiert werden kann und/oder eine Tachykardiedetektion durchgeführt werden kann.

Zur Erläuterung der Erfindung wird im folgenden auf die Figuren der Zeichnung bezug genommen; im einzelnen zeigen

FIG. 1     den Verlauf des Elektrokardiogramms und den zugehörigen Verlauf des Ventrikelvolumens bei einem Frequenzwechsel,

FIG. 2     das Blockschaltbild eines Herzschrittmachers, bei dem mit Hilfe des Frequenzwechsels ein belastungsabhängiger Meßparameter erfaßt und zur Frequenzsteuerung des Herzschrittmachers herangezogen wird,

FIG. 3     in einem Diagramm den Verlauf des Schlagvolumens eines normalen Herzens in Abhängigkeit von der Herzschlagfrequenz bei unterschiedlichen Belastungsstufen,

FIG. 4     das Blockschaltbild eines Herzschrittmachers mit hämodynamischer Optimierung der Stimulationsfrequenz und

FIG. 5     das Blockschaltbild eines Herzschrittmachers mit Mitteln zur Erkennung tachykarder Zustände des Herzens

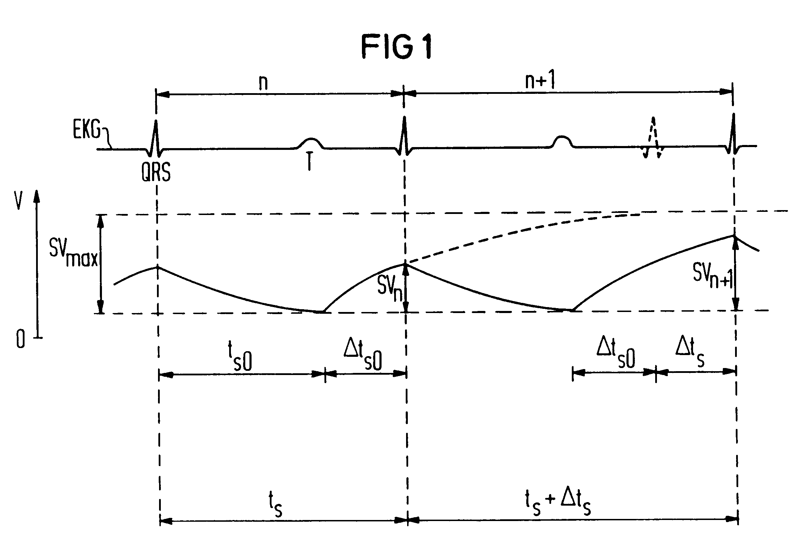

FIG 1 zeigt im oberen Teil den Signalverlauf eines Elektrokardiogramms EKG über zwei aufeinander folgende Herzzyklen n und n + 1 und darunter den dazugehörigen Verlauf des Volumens V der beiden Ventrikel eines Herzens. Der Verlauf des Ventrikelvolumens V ist hier durch exponentiell verlaufende Kurvenabschnitte vereinfacht dargestellt. Beim Auftreten des den Herzzyklus n einleitenden QRS-Komplexes im EKG beginnt die systolische Phase, in der sich der Herzmuskel zunächst anspannt und anschließend bis auf ein Restvolumen zusammenzieht, wobei das Blut aus den Ventrikeln ausgetrieben wird. Die Länge der systolischen Phase ist hier

mit $t_{so}$ bezeichnet. Am Ende der T-Welle des EKGs beginnt die diastolische Phase, in der sich die Ventvikel entspannen und sich anschließend bei exponentiell verlaufender Vergrößerung des Ventrikelvolumens V wieder füllen. Die Dauer der diastolischen Phase, die durch den nächstfolgenden Herzschlag beendet wird, ist mit $\Delta t_{so}$ bezeichnet, so daß für die Dauer $t_s$ des mit n bezeichneten Herzzyklus gilt: $t_s = t_{so} + \Delta t_{so}$.

Das Schlagvolumen $SV_n$ des ersten Herzzyklus n kann, wie FIG 1 verdeutlicht, näherungsweise durch die Gleichung

$$SV_n = SV_{max} \cdot (1 - \exp - (\Delta t_{so}/T))$$

beschrieben werden, wobei $SV_{max}$ das sich aufgrund der gegebenen Belastung des Patienten bei einer maximalen Dauer des Herzzyklus ergebende Schlagvolumen ist und wobei T die von der Kontraktilität des Herzmuskels abhängige Zeitkonstante für den Anstieg des Ventrikelvolumens V in der diastolischen Phase ist. In die oben stehende Gleichung für das Schlagvolumen $SV_n$ gehen zwei Parameter, nämlich $SV_{max}$ und T ein, die sich durch Änderung der Kontraktilität des Herzmuskels in Abhängigkeit von der physischen und psychischen Belastung des Patienten ändern. Es ist also grundsätzlich möglich, über eine meßtechnische Erfassung des Schlagvolumens SV, wie z.B. die Messung von Änderungen $\Delta Z$ der elektrischen Gewebeimpedanz Z im Bereich des Herzens den Belastungszustand des Patienten zu erfassen und beispielsweise zur Frequenzsteuerung eines Herzschrittmachers heranzuziehen. Dabei wird davon ausgegangen, daß die Impedanzschwankungen $\Delta Z$ proportional zum Schlagvolumen SV sind. Allerdings ist der schlagvolumenabhängige Meßparameter, hier also das Impedanzsignal, einer Vielzahl von Störeinflüssen wie z.B. der Atemtätigkeit des Patienten, Bewegungsartefakten, Drifterscheinungen, Sensitivitäts- und Linearitätsänderungen ausgesetzt, die nicht mit der Herztätigkeit korrelieren. Zur Eliminierung dieser Störeinflüsse wird, wie im folgenden gezeigt wird, der Meßparameter in Abhängigkeit von der Änderung der Dauer aufeinanderfolgender Herzzyklen n und n + 1 bewertet.

Wie FIG. 1 zeigt, ist die Dauer des Herzzyklus n + 1 gegenüber dem vorangehenden Zyklus n um $\Delta t_s$ verlängert, so daß bei unveränderter Dauer der Systole die diastolische Phase des zweiten Herzzyklus n + 1 um denselben Berag $\Delta t_s$ verlängert ist. Bei unveränderter Belastung des Patienten ergibt sich somit für das Schlagvolumen $SV_{n+1}$ des zweiten Herzzyklus n + 1

$$SV_{n+1} = SV_{max} (1 - \exp - (\Delta t_{so} + \Delta t_s)/T).$$

Damit ergibt sich für die Änderung $\Delta$ SV(+) des Schlagvolumens SV aufgrund der Vergrößerung des Abstands aufeinanderfolgender Herzschläge um den Betrag $+\Delta t_s$:

$$\Delta SV(+) = SV_{n+1} - SV_n = SV_{max} \exp - (\Delta t_{so}/T)\cdot - (1 - \exp - (\Delta t_s/T)).$$

Bei einer Verringerung der Herzzyklusdauer, also einer Änderung des Abstandes zwischen zwei Herzschlägen um $-\Delta t_s$ ergibt sich entsprechend für die Änderung $\Delta$ SV(-) des Schlagvolumens SV:

$$\Delta SV(-) = SV_{max} \exp - (\Delta t_{so}/T)\cdot(1 - \exp (\Delta t_s/T)).$$

Das Verhältnis der beiden Schlagvolumenänderungen ergibt sich damit zu

$$\Delta SV(+)/\Delta SV(-) = - \exp - (\Delta t_s/T)$$

und enthält nur noch die belastungsabhängige Zeitkonstante T ohne die störungsbehaftete Größe $SV_{max}$.

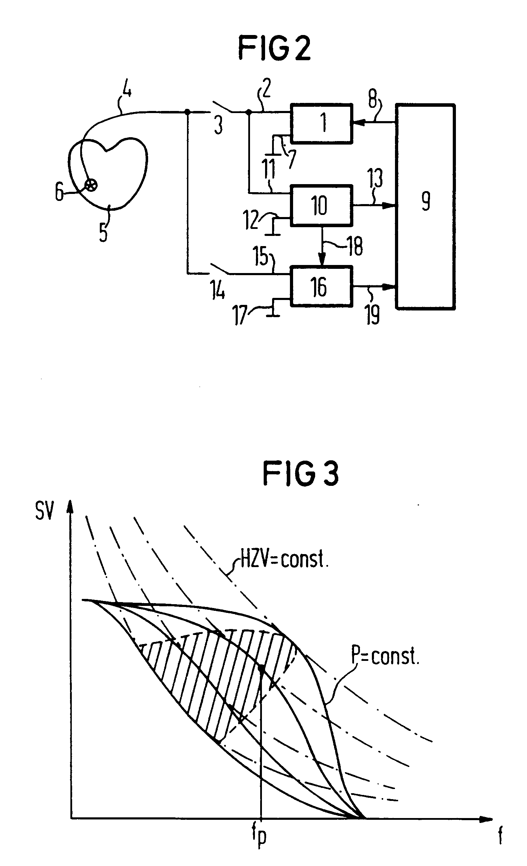

FIG. 2 zeigt das Blockschaltbild eines Herzschrittmachers, bei dem die oben beschriebene Erfassung eines belastungsabhängigen Meßparameters zur Steuerung der Stimulationsfrequenz herangezogen wird. Der Herzschrittmacher enthält einen Stimulationsimpulsgenerator 1, der an einem Ausgangsanschluß 2 über einen steuerbaren Schalter 3 und eine Elektrodenleitung 4 mit einer im Herzen 5 des Patienten plazierten Elektrode 6 verbunden ist. Der zweite Ausgangsanschluß 7 des Stimulationsimpulsgenerators 1 ist mit dem hier nicht gezeigten Gehäuse des Herzschrittmachers, das als Bezugselektrode dient, verbunden. Der Stimulationsimpulsgenerator 1 ist über eine Steuerleitung 8 mit einer Herzschrittmachersteuerung 9 verbunden, die über die Steuerleitung 8 die Abgabe von Stimulationsimpulsen durch den Stimulationsimpulsgenerator 1 veranlaßt. Ein Herzschlagdetektor 10 ist zur Detektion von stimulierten oder natürlichen Herzschlägen zwischen der Elektrode 6 und dem Herzschrittmachergehäuse mit einem ersten Eingangsanschluß 11 an dem Ausgangsanschluß 2 des Stimulationsimpulsgenerators 1 und mit einem zweiten Eingangsanschluß 12 an dem Herzschrittmachergehäuse angeschlossen; ausgangsseitig ist der Herschlagdetektor 10 über eine Signalleitung 13 mit der Herzschrittmachersteuerung 9 verbunden. Die Elektrodenleitung 4 ist über einen weiteren steuerbaren Schalter 14 mit dem ersten Eingangsanschluß 15 eines Meßparameteraufnehmers 16 verbunden, der bei dem gezeigten Ausführungsbeispiel als Meßparameter die Gewebeimpedanz zwischen der Elektrode 6 und dem Herzschrittmachergehäuse erfaßt, an dem zu diesem Zweck der zweite Eingansanschluß 17 des Meßparameteraufnehmers 16 angeschlossen ist. Der Meßparameteraufnehmer 16 ist über eine Steuerleitung 18 mit dem Herzschlagdetektor 10 und über eine Ausgangssignalleitung 19 mit der Herzschrittmachersteuerung 9 verbunden. Die steuerbaren Schalter 3 und 14 dienen zur Entkopplung des Stimulationsimpulsgenerators 1 und Herzschlagdetektors 10 einerseits und des Meßparameteraufnehmers 16 andererseits, so daß sich diese nicht gegenseitig beeinflussen können. Bei dem gezeigten Ausführungsbeispiel erfolgt die Impedanzmessung zwischen der Elektrode 6 und dem Herzschrittmachergehäuse; es ist aber auch ein Mehrelektrodensystem denkbar, bei dem unterschiedliche Elektroden für die Stimulation, Herzschlagdetektion und Impedanzmessung vorgesehen sind.

Die Funktionsweise des gezeigten Herzschrittmachers ist wie folgt. Die Herzschrittmachersteuerung 9 gibt eine bestimmte, z.B. von außen her programmierbare Frequenz f vor, mit der der Stimulaticnsimpulsgenerator 1 zur Abgabe von Stimulationsimpulsen an das Herz 5 veranlaßt wird. Der Frequenz f entspricht der Abstand $t_s$ der einzelnen Stimulationsimpulse mit $t_s = 1/f$. Mit dem Herzschlagdetektor 10 werden sowohl natürliche, als auch stimulierte Herzschläge detektiert. Mit jedem detektierten Herzschlag wird in der Herzschrittmachersteuerung 9 ein Zeitintervall der Dauer $t_s$ gestartet, nach dessen vollständigem Ablauf die Abgabe eines Stimulationsimpulses veranlaßt wird und das Zeitintervall neu gestartet wird. Wird vor Ablauf dieses Zeitintervalles ein natürlicher Herzschlag detektiert, so wird das Zeitintervall ohne Erzeugung eines Stimulationsimpulses neu gestartet.

Im Abstand von mehreren Sekunden wird in der Herzschrittmachersteuerung 9 der Abstand zwischen zwei aufeinanderfolgenden Stimulationsimpulsen abwechselnd um den Betrag $+\Delta t_s$ und den Betrag $-\Delta t_s$ verändert. Diese vereinzelten Änderungen führen jeweils zu einer sofortigen Reaktion des Schlagvolumens SV wegen dor Änderung der diastolischen Füllphase, während der allgemeine Druck im Kreislaufsystem sich nicht ändert und damit das mittlere Herzzeitvolumen unverändert bleibt. Den Änderungen $\Delta$ SV(+) und $\Delta$ SV(-) des Schlagvolumens entsprechen Änderungen $\Delta$ ($\Delta$ Z(+)) und $\Delta$ ($\Delta$ Z(-)) der Impedanzschwankungen $\Delta$ Z. Die Erfassung der Impedanz mittels des Meßparameteraufnehmers 16 wird über die Steuerleitung 18 mit den detektierten Herzschlägen synchronisiert. Bei einer Verlängerung des Stimulationsimpulsabstandes um $\Delta t_s$ wird also zunächst in dem vorangehenden Herzzyklus n die Impedanzschwankung $\Delta Z_n$ und danach in dem verlängerten Nerzzyklus n+1 die Impedanzschwankung $\Delta Z_{n+1}$ erfaßt; anschließend wird die Differenz beider Impedanzschwankungen mit $\Delta$ ($\Delta$ Z(+)) = $\Delta Z_{n+1}$ -$\Delta$

$Z_n$ gebildet. Auf dieselbe Weise wird bei einer Verkürzung des Stimulationsimpulsabstandes um $\Delta t_s$ die Impedanzschwankungsänderung $\Delta (\Delta Z(-))$ ermittelt. Von diesen beiden Werten wird in der Herzschrittmachersteuerung 9 der Quotient $\Delta (\Delta Z(+))/\Delta(\Delta Z(-))$ gebildet, der dem Verhältnis der durch den Stimulationsfrequenzwechsel hervorgerufenen Schlagvolumenänderungen $\Delta SV(+)/\Delta SV(-) = - \exp - (\Delta t_s/T)$ entspricht und somit, wie oben bereits gezeigt, ein Maß für den Belastungszustand des Patienten darstellt. Der so ermittelte Istwert für die Belastung wird in der Herzschrittmachersteuerung 9 mit einem Sollwert verglichen, der entweder als konstanter Wert durch eine hier nicht gezeigte äußere programmiereinheit vorgeben werden kann, oder bei dem es sich um einen von der Frequenz f abhängigen Wert handeln kann. Aufgrund des Soll-Istwert -Vergleichs wird die Frequenz f, mit der die Herzschrittmachersteuerung 9 den Stimulationsimpulsgeber 1 zur Abgabe von Stimulationsimpulsen veranlaßt, gesteuert.

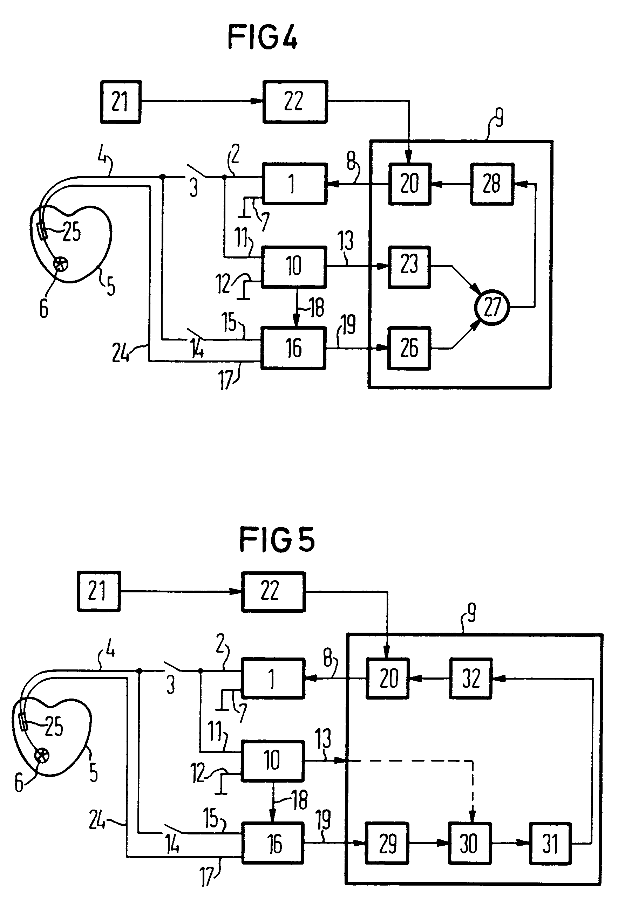

Im folgenden wird anhand von FIG. 3 und FIG. 4 ein Ausführungsbeispiel im Rahmen der Erfindung zur hämodynamischen Frequenzoptimierung bei einem Herzschrittmacher erläutert. Die körpereigene Kreislaufregelung regelt das Herzzeitvolumen HZV in Abhängigkeit von der physischen und psychischen Belastung des Patienten. Das Herzzeitvolumen HZV als Produkt aus der Herzschlagfrequenz f und dem Schlagvolumen SV ist dabei abhängig von der Druckdifferenz $\Delta p$ und dem peripheren Strömungswiderstand R, d.h.: $HZV = SV \cdot f = \Delta p/R$. Dabei reagiert der periphere Strömungswiderstand R weitgehend autonom auf die Belastung, indem beispielsweise bei der Aktivierung eines Muskels dessen Blutgefäße sich erweitern und somit eine Reduzierung des peripheren Strömungswiderstandes R bewirken. Der dadurch verursachte kurzfristige Druckabfall wird von entsprechenden Rezeptoren des zentralen Nervensystems aufgenommen und in ein Signal an den Herzmuskel zur Erhöhung des Herzzeitvolumens HZV über das Schlagvolumen SV und die Herzschlagfrequenz f umgewandelt. Das Herzzeitvolumen HZV stabilisiert sich dann im Mittel auf einen der Belastung proportionalen Wert.

FIG. 3 zeigt in einem Diagramm für ein normales Herz den Verlauf des Schlagvolumens SV in Abhängigkeit von der Herzschlagfrequenz f bei unterschiedlichen Belastungsstufen P. Die Linien konstanten Herzzeitvolumens HZV sind strichpunktiert eingezeichnet. Wie das Diagramm zeigt, gibt es für jeden Belastungszustand P einen Frequenzwert $f_p$, ab dem eine weitere Erhöhung der Herschlagfrequenz f keine weitere Erhöhung des Herzzeitvolumens HZV ergibt. In dem Diagramm ist der Bereich, in dem die eine Änderung der Frequenz f so gut wie keine Änderung des Herzzeitvolumens HZV ergibt, schraffiert eingezeichnet. Die große individuelle Schwankungsbreite der kardialen Leistungsfähigkeit macht es für Herzschrittmacherpatienten erforderlich, die Anpassung der Stimulationsfrequenz an die Belastungssituation derart zu optimieren, daß sich das Herzzeitvolumen HZV proportional der Belastung anpaßt und eine Frequenzerhöhung nicht zu einer Erniedrigung des Herzzeitvolumens HZV führt.

Bei der folgenden Erläuterung eines Beispiels zur hämodynamischen Frequenzoptimierung bei einem Herzschrittmacher wird der oben angegebene Tatbestand ausgenutzt, daß die körpereigene Kreislaufregelung bei gleichbleibender Belastungssituation das Herzvolumen HZV annähernd konstant hält, indem ab einem belastungsabhängigen, individuell typischen Frequenzwert fp Erhöhungen $\Delta f$ der Stimulationsfrequenz f durch eine entsprechende Erniedrigung $-\Delta SV$ des Schlagvolumens SV kompensiert werden; d.h.: bei konstanter Belastung gilt für f größer fp:

$$HMV = SV \cdot f = (SV - \Delta SV) \cdot (f + \Delta f)$$

Damit ergibt sich für die relative Änderung des Schlagvolumens SV die Beziehung:

$$\Delta SV/SV = \Delta f/(f + \Delta f).$$

Wie FIG. 1 zeigt, führen Verkürzungen des Impulsabstandes $t_s$ um den Betrag $\Delta t_{so}$ dazu, daß in dem zweiten Herzzyklus $n+1$ das Schlagvolumen SV zu Null wird. Dann gilt für die Änderung $\Delta SV_o$ des Schlagvolumens SV:

$$\Delta SV_o = SV_{n+1} - SV_n = O - SV_n.$$

Bei einer gegebenen Stimulationsfrequenz f läßt sich demnach das Schlagvolumen SV dadurch bestimmen, daß mehrmals in Zeitabständen von einigen Sekunden der Abstand zwischen zwei, den Herzzyklus $n+1$ definierenden Stimulationsimpulsen um einen jedesmal größeren Betrag $\Delta t_s$ verkürzt wird und die dadurch hervorgerufene Änderung $\Delta SV$ des Schlagvolumens SV erfaßt wird. Dieser Vorgang wird solange fortgesetzt, so lange $\Delta SV$ dabei größer wird. Sobald jedoch für $\Delta SV$ ein gegenüber dem vorangegangenen Wert kleinerer Wert ermittelt wird, wird der vorangegangene, d.h. maximale Wert für $\Delta SV$ als $\Delta SV_o = -SV_n$ definiert und der Betrag $\Delta t_s$, um den der Impulsabstand $t_s$ verkürzt wurde, als $\Delta t_{so}$ definiert. Wie schon bei dem vorangegangenen Ausführungsbeispiel gezeigt, können die Änderungen $\Delta SV$ bzw. $\Delta SV_o$ des Schlagvolumens SV über eine Impedanzmessung im Bereich des Herzens erfaßt werden. Da die Impedanzschwankungen $\Delta Z$ eine Funktion des Schlagvolumens SV sind, kann die oben geforderte Bestimmung von $\Delta SV/SV = - \Delta SV/\Delta SV_o$ durch das Verhältnis der Änderungen der Impedanzschwankungen $\Delta (\Delta Z)/\Delta (\Delta Z_o)$ ermittelt werden, wobei $\Delta Z_o$ die Impedanzschwankungen bei einer Verlängerung des StimulationsimpulsabstandeS um $\Delta t_s = \Delta t_{so}$ sind.

FIG. 4 zeigt das Blockschaltbild eines Herzschrittmachers, bei dem die oben beschriebene hämodynamische Frequenzoptimierung Anwendung findet; dabei sind gleiche Funktionsblöcke wie in FIG. 2 mit denselben Bezugszeichen versehen. Der Herzschrittmacher enthält einen Stimulationsimpulsgenerator 1, der an einem ersten Ausgangsanschluß 2 über einen steuerbaren Schalter 3 und eine erste Elektrodenleitung 4 mit einer im Herzen 5 des Patienten plazierten distalen Spitzenelektrode 6 verbunden ist. Der zweite Ausgangsanschluß 7 des Stimulationsimpulsgenerators 1 ist mit dem hier nicht gezeigten Gehäuse des Herzschrittmachers verbunden. Der Stimulationsimpulsgenerator 1 ist über eine Steuerleitung 8 mit einer Frequenzsteuereinheit 20 verbunden, die Bestandteil einer Herzschrittmachersteuerung 9 ist und den Stimulationspulsgenerator 1 zur Abgabe von Stimulationsimpulsen mit einer vorgegebenen Frequenz steuert. Die Frequenz ist in Abhängigkeit von dem Ausgangssignal eines den Belastungszustand des Patienten detektierenden Sensors 21 steuerbar, dessen Ausgangssignal über eine Signalauswerteeinrichtung 22 der Frequenzsteuereinheit 20 zugeführt wird. Zur Detektion von natürlichen und stimulierten Herzschlägen zwischen der Spitzenelektrode 6 und dem Gehäuse des Herzschrittmachers ist ein Herzschlagdetektor 10 mit einem ersten Eingangsanschluß 11 an dem Ausgangsanschluß 2 des Stimulationsimpulsgerators 1 und mit einem zweiten Eingangsanschluß 12 an dem Herzschrittmachergehäuse angeschlossen. Ausgangsseitig ist der Herzschlagdetektor 10 über eine Signalleitung 13 mit einem Differenz- und Quotientenbildner 23 in der Herzschrittmachersteuerung 9 verbunden. Ein Meßparameteraufnehmer 16 ist an seinem ersten Eingangsanschluß 15 über einen weiteren steuerbaren Schalter 14 und die Elektrodenleitung 4 mit der Spitzenelektrode 6 und an seinem zweiten Eingangsanschluß 17 über eine zweite Elektrodenleitung 24 mit einer von der ersten Elektrode 6 beabstandeten Ringelektrode 25 verbunden. Der Meßparameteraufnehmer 16 dient zur Erfassung der Impedanz zwischen den beiden Elektroden 6 und 25. Der Meßparameteraufnehmer 16 ist ferner über eine Steuerleitung 18 mit dem Herzschlagdetektor 10 und über eine Ausgangssignalleitung 19 mit einer Signalbehandlungsstufe 26 der Herzschrittmachersteuerung 9 verbunden. Der Differenz- und Quotientenbildner 23 und die Signalbehandlungsstufe 26 sind ausgangsseitig an einem Differenzvergleicher 27 angeschlossen, dessen Ausgangssignal einer die Frequenzsteuereinheit 20 steuernden Regelstufe 28 zugeführt ist.

Die Frequenzsteuereinheit 20 veranlaßt den Stimulationsimpulsgeber 1 zur Abgabe von Stimulationsimpulsen mit einer von dem mit dem Sensor erfaßten Belastungszustand des Patienten abhängigen Frequenz. Dabei wird im Abstand von einigen Sekunden der Abstand zwischen zwei aufeinanderfolgenden Stimulationsimpulsen einmalig um den Betrag $\Delta t_s$ verkürzt. In dem Meßparameteraufnehmer 16 wird bei einem jeden derartigen Frequenzwechsel die Impedanz im Herzen 5 zwischen den Elektroden 6 und 25 synchron zu den von dem Herzschlagdetektor 10 detektierten Herzschlägen erfaßt und in der Signalbehandlungsstufe 26 die Differenz $\Delta(\Delta Z)$ zwischen den Impedanzschwankungen $\Delta Z_n$ bei dem der Änderung vorangehenden Herzzyklus n und den Impedanzschwankungen $\Delta Z_{n+1}$ bei dem Herzzyklus n + 1 mit der veränderten Dauer gebildet. Der Vorgang der einmaligen Impulsabstandsverringerung wird im Abstand von einigen Sekunden mit einer immer größeren Verkürzung $-\Delta t_s$ wiederholt und dabei in der Signalbehandlungsstufe 26 der jeweils neue Wert für $\Delta (\Delta Z)$ mit dem zuvor ermittelten Wert verglichen; ist der neu ermittelte Wert größer als der vorangegangene Wert, wird der Vorgang der Impulsabstandsverringerung so lange fortgesetzt, bis der neu ermittelte Wert für $\Delta (\Delta Z)$ um einen vorgegebenen Betrag kleiner als der zuvor ermittelte Wert ist. Dann wird der zuvor ermittelte Wert für $\Delta(\Delta Z)$ als $\Delta (\Delta Z_o)$ definiert und gespeichert.

Ändert die Frequenzsteuereinheit 20 die Stimulationsfrequenz f über längere Zeitabstände (Minuten), weil entweder der Sensor 21 eine Belastungsänderung detektiert oder weil die Frequenzsteuereinheit 20 selbstätig eine Frequenzoptimierung durchführt, dann wird der dadurch bedingte neue Wert von $\Delta (\Delta Z_o)_{m+1}$ in der Signalbehandlungsstufe 26 mit dem vor der längerfristigen Frequenzänderung $\Delta f$ zuletzt ermittelten Wert $\Delta (\Delta Z_o)_m$ verglichen und die Differenz $\Delta(\Delta (\Delta Z_o))$ beider Werte gebildet; anschließend wird der Quotient $\Delta (\Delta (\Delta Z_o))/\Delta (\Delta Z_o)_m$ gebildet, der der relativen Änderung $\Delta SV/SV$ des Schlagvolumens SV aufgrund der Frequenzänderung $\Delta f$ entspricht. In dem Differenz- und Quotientenbildner 23 wird aufgrund des zeitlichen Auftretens der detektierten Herzschläge der Quotient $\Delta f/(f + \Delta f)$ gebildet. In dem Differenzvergleicher 27 wird durch Vergleich der Ausgangswerte der Funktionsblöcke 23 und 26 entschieden, ob der Quotient $\Delta (\Delta (\Delta Z_o))/ \Delta (\Delta Z_o)_m$, d.h. $\Delta SV/SV$ größer oder kleiner als Quotient $\Delta f/(f + \Delta f)$ ist und damit, ob die Frequenzänderung $\Delta f$ zu einer Verbesserung oder Verschlechterung des Herzzeitvolumens HMV = SV•f geführt hat. In Abhängigkeit von dem Vergleichsergebnis wird die Frequenzsteuereinheit 20 über die Regelstufe 28 so gesteuert, daß Frequenzänderungen Δ f, die bei konstanter Belastung eine Verschlechterung des Herzzeitvolumens HZV bewirken, zurückgenommen werden.

Im folgenden wird schließlich ein Ausführungsbeispiel zur Erkennung tachykarder Zustände des Herzens beschrieben. Über die Erfassung des Schlagvolumens SV läßt sich prinzipiell die Effektivität des Herzmuskels bewerten und speziell bei hohen Frequenzen f feststellen, ob die Pulsaktionen des Herzmuskels ineffektiv, d.h. tachykard sind. Die Analyse schlagvolumenabhängiger Meßparameter mit Hilfe kurzer Frequenzwechsel durch Änderung des Impulsabstandes aufeinanderfolgender Stimulationsimpulse ermöglicht die Detektion derartiger tachykarder Zustände. Dazu wird im Rahmen der Erfindung die mit Hilfe eines Herzschlagdetektors detektierte Herzschlagfrequenz durch kurze Frequenzanhebungen $\Delta f_1, \Delta f_2$, ..., d.h. kurze Verringerungen $\Delta t_{s1}$, $\Delta t_{s2}$, ... des Abstands $\Delta t_s$ aufeinanderfolgender Stimulationsimpulse, moduliert und durch Differenz- und Quotientenbildung bewertet, ob und wie stark sich der schlagvolumenabhängige Meßparameter in Abhängigkeit von den Frequenzwechseln $\Delta f_1, \Delta f_2$, ... geändert hat. Liegt die Änderung des schlagvolumenabhängigen Meßparameters unter einer vorgegebenen Schwelle, so wird ein tachykarder Zustand detektiert und angezeigt.

Eine verbesserte Analyse kann erreicht werden, indem für mehrere hohe Grundfrequenzen $f_1$, $f_2$, ... ein von Änderungen $\Delta f_{11}$, $\Delta f_{12}$, ..., $\Delta f_{21}$, $\Delta f_{22}$, ... der Grundfrequenzen abhängiges Änderungsmuster des schlagvolumenabhängigen Meßparameters erstellt und gespeichert wird und für eine Vergleichsanalyse zur Detektion einer Tachykardie benutzt wird.

FIG 5 zeigt ein entsprechendes Ausführungsbeispiel für die Tachykardiedetektion bei einem Herzschrittmacher, wobei im Vergleich zu dem in FIG. 4 gezeigten Herzschrittmacher nur Unterschiede im Aufbau der Herzschrittmachersteuerung 9 bestehen. In der dem Meßparameteraufnehmer 16 nachgeordneten Auswerteschaltung 29 werden die aufgrund von Änderungen $\Delta t_s$ des Stimulationsimpulsabstandes $t_s$ bei zwei aufeinanderfolgenden Herzzyklen n und n + 1 erfolgten Änderungen $\Delta$ ($\Delta$ Z) der Impedanzschwankungen $\Delta$ Z erfaßt und durch Quotientenbildung ebenso wie bei dem Ausführungsbeispiel nach FIG. 2 die störbehaftete Größe $SV_{max}$ eliminiert.Die damit bestimmten Werte werden zugehörig zu der von dem Herzschlagdetektor 10 detektierten Herzschlagfrequenz f in einem Speicher 30 festgehalten. Die so gespeicherten Werte werden in einer weiteren Auswertestufe 31 zur Detektion einer Tachykardie mit vorgegebenen, programmierbaren Werten oder mit einem vorher von dem Herzschrittmacher erstellten Wertemuster verglichen. Bei Detektion einer Tachykardie wird eine Funktionseinheit 32 zur Steuerung der Frequenzsteuereinheit 20 im Sinne einer Beendigung der Tachykardie aktiviert.

## Patentansprüche

1. Anordnung, insbesondere Herzschrittmacher, mit einer Meßeinrichtung zur Erfassung eines Meßparameters (M) der Herzaktivität, und Schaltmittel, die bei einer einmaligen oder vereinzelt auftretenden Änderung der Frequenz (f) bzw. Dauer ($t_s$) eines Herzzyklus (n + 1) im Vergleich zum vorangegangenen Herzzyklus (n) den Grad einer daraus resultierenden Änderung des Meßparameters (M) in Abhängigkeit von dem Grad der Frequenzänderung ( f) bewerten, **dadurch gekennzeichnet,** daß eine Steuerung (9) den Abstand zwischen zwei aufeinanderfolgenden Stimulationsimpulsen verändert, wobei diese Veränderungen, die zu einer sofortigen Reaktion des Meßparamters (M) führen, in so einem Abstand ausgeführt werden, daß der allgemeine Druck im Kreislaufsystem sich nicht ändert.

2. Anordnung nach Anspruch 1, **dadurch gekennzeichnet**, daß als Grad der Änderung des Meßparameters (M) die Differenz ($\Delta$ M) von in zwei aufeinanderfolgenden Meßzyklen (n, n + 1) erfaßten Meßwerten ($M_n$) und ($M_{n+1}$) bestimmt wird.

3. Anordnung nach Anspruch 1 oder 2, **dadurch gekennzeichnet**, daß Parameteränderungen ($\Delta M$) bewertet werden, die durch von einer Frequenzsteuereinheit (20) gesteuerte definierte Änderungen der Stimulationsfrequenz ($\Delta f$) hervorgerufen werden.

4. Anordnung nach einem der Ansprüche 1 bis 3, **dadurch gekennzeichnet**, daß zur Bewertung der Quotient zweier Meßparameteränderungen ($\Delta M1$) und ($\Delta M2$) berechnet wird, die abhängig von unterschiedlichen Pulsabstandsänderungen ($\Delta t_{s1}$) und ($\Delta t_{s2}$) gemessen werden.

5. Anordnung nach einem der vorangehenden Ansprüche, **dadurch gekennzeichnet**, daß mehrere Bewertungen bei verschiedenen Grundfrequenzen ($f_1$, $f_2$, ...) durchgeführt werden, die über einen längeren Zeitraum bei konstanter Belastung konstant sind, und daß diese Werte frequenzbezogen gespeichert werden.

6. Anordnung nach einem der Ansprüche 1 bis 4, **dadurch gekennzeichnet**, daß die Quotienten

von Änderungen belastungsabhängiger Meßparameter durch Vergleich mit einem gegebenenfalls frequenzabhängigen Sollwert zur Regelung der Stimulationsfrequenz benutzt werden.

7. Anordnung nach einem der Ansprüche 1 bis 4, **dadurch gekennzeichnet**, daß die Differenzwerte ( M) eines schlagvolumenabhängigen Meßwertes (M) bei steigendem Wert der Frequenzänderungen (Δf) derart ausgewertet werden, daß eine maximale Meßwertänderung ($\Delta M_{max}$) bei $\Delta f_o = 1/\Delta t_{so}$ detektiert wird.

8. Anordnung nach Anspruch 6, **dadurch gekennzeichnet**, daß die Differenz von zwei maximalen Meßwertänderungswerten bei zwei unterschiedlichen Grundfrequenzen (f1) und (f2) aber gleicher Belastung bezogen auf den Wert ($\Delta M_{max}$) bei der Frequenz (f1) mit dem Quotienten Δf/(f + f) verglichen werden und daß der Vergleichswert zur hämodynamischen Optimierung der Frequenzregelung genutzt wird.

9. Anordnung nach einem der Ansprüche 1 bis 4, **dadurch gekennzeichnet**, daß Quotienten verschiedener kurzer Änderungen schlagvolumenabhängiger Meßparameter bei gleicher Grundfrequenz mit gespeicherten Quotienten-Sollwerten für diese Frequenz verglichen werden und daß damit die Effektivität des Herzmuskels zur Tachykardiedetektion analysiert wird.

10. Anordnung nach Anspruch 1 oder 2, **dadurch gekennzeichnet**, daß die Differenzen von kurzfristigen Änderungen frequenz- und belastungsabhängiger Meßparameter während gleichbleibender Belastung mit mittelfristigen Änderungen dieser Parameter nach einer Belastungsänderung verglichen werden und zur Trennung der Frequenz- von der Belastungsabhängigkeit dieser Parameter verwendet werden.

**Claims**

1. Arrangement, in particular heart pacemaker, having a measuring device for recording a heart activity measurement parameter (M) and switching means which, in the event of a once-only or sporadically occurring change of the frequency (f) or duration ($t_s$) of a heart cycle (n + 1) in comparison with the preceding heart cycle (n), assess the degree of a change, resulting therefrom, of the measurement parameter (M) as a function of the degree of the frequency change (f), characterized in that a control system (9) changes the spacing between two successive stimulation pulses, these changes, which lead to an immediate reaction of the measurement parameter (M), being executed at such a spacing that the general pressure in the circulatory system does not change.

2. Arrangement according to Claim 1, characterized in that the difference (ΔM) of measured values ($M_n$) and ($M_{n+1}$) recorded in two successive measurement cycles (n, n + 1) is determined as degree of the change of the measurement parameter (M).

3. Arrangement according to Claim 1 or 2, characterized in that parameter changes (ΔM) which are generated by defined changes of the stimulation frequency (Δf) controlled by a frequency control unit (20) are assessed.

4. Arrangement according to one of Claims 1 to 3, characterized in that for the assessment the quotient is computed of two measurement parameter changes (ΔM1) and (ΔM2), which are measured as a function of differing pulse spacing changes ($\Delta t_{s1}$) and ($\Delta t_{s2}$).

5. Arrangement according to one of the preceding claims, characterized in that a plurality of assessments are carried out at differing base frequencies ($f_1$, $f_2$, ...) which are constant over a relatively long period of time at constant loading, and in that these values are stored in frequency-related form.

6. Arrangement according to one of Claims 1 to 4, characterized in that the quotients of changes of loading-dependent measurement parameters are used for the regulation of the stimulation frequency by comparison with a possibly frequency-dependent theoretical value.

7. Arrangement according to one of Claims 1 to 4, characterized in that the difference values (M) of a beat-volume-dependent measured value (M) are evaluated as the value of the frequency changes (Δf) increases, in such a manner that a maximum measured value change ($\Delta M_{max}$) is detected at $\Delta f_o = 1/\Delta t_{so}$.

8. Arrangement according to Claim 6, characterized in that the difference of two maximum measured value change values at two different base frequencies (f1) and (f2) but the same loading related to the value ($\Delta M_{max}$) at the frequency (f1) is compared with the quotient

$\Delta f/(f + f)$, and in that the comparison value is used for the haemodynamic optimization of the frequency regulation.

9. Arrangement according to one of Claims 1 to 4, characterized in that quotients of differing short changes of beat-volume-dependent measurement parameters at the same base frequency are compared with stored quotient theoretical values for this frequency, and in that the effectiveness of the heart muscle is thus analysed for tachycardial detection.

10. Arrangement according to Claim 1 or 2, characterized in that the differences of short-term changes of frequency-dependent and loading-dependent measurement parameters during constant loading are compared with medium-term changes of these parameters after a loading change and are used for the separation of the frequency dependence from the loading dependence of these parameters.

**Revendications**

1. Dispositif, notamment stimulateur cardiaque, comportant un dispositif de mesure servant à détecter un paramètre de mesure (M) de l'activité cardiaque, et des moyens de commutation, qui, dans le cas d'une modification, qui apparaît une fois ou isolément, de la fréquence (f) ou de la durée ($t_s$) d'un cycle cardiaque (n + 1) par rapport au cycle cardiaque précédent (n), évaluent le degré de la variation, qui en résulte, du paramètre de mesure (M) en fonction du degré de variation de la fréquence (f), caractérisé par le fait qu'une unité de commande (9) modifie la distance entre deux impulsions de stimulation successives, ces modifications, qui conduisent à une réaction immédiate du paramètre de mesure (M), étant exécutées à une distance telle que la pression générale dans le système de la circulation ne varie pas.

2. Dispositif suivant la revendication 1, caractérisé par le fait qu'on détermine, en tant que degré de la modification du paramètre de mesure (M), la différence ($\Delta M$) entre des valeurs de mesure ($M_n$) et ($M_{n+1}$), détectées pendant deux cycles de mesure successifs (n, n + 1).

3. Dispositif suivant la revendication 1 ou 2, caractérisé par le fait qu'on évalue des variations ($\Delta M$) du paramètre, qui sont provoquées par des modifications définies de la fréquence de stimulation ($\Delta f$), qui sont commandées par une unité (20) de commande de la fréquence.

4. Dispositif suivant l'une des revendications 1 à 3, caractérisé par le fait que pour l'évaluation, on calcule le quotient de deux variations ($\Delta M1$) et ($\Delta M2$) du paramètre de mesure, que l'on mesure en fonction de variations différentes ($\Delta t_{s1}$) et ($\Delta t_{s2}$) de la distance entre impulsions.

5. Dispositif suivant l'une des revendications précédentes, caractérisé par le fait qu'on exécute plusieurs évaluations pour différentes fréquences de base ($f_1$, $f_2$, ...), qui sont constantes sur un intervalle de temps assez long, pour une charge constante, et qu'on mémorise ces valeurs d'une manière rapportée à la fréquence.

6. Dispositif suivant l'une des revendications 1 à 4, caractérisé par le fait qu'on utilise les quotients de variations de paramètres de mesure, qui dépendent de la charge, par comparaison à une valeur de consigne, qui dépend éventuellement de la fréquence, pour régler la fréquence de stimulation.

7. Dispositif suivant l'une des revendications 1 à 4, caractérisé par le fait qu'on évalue les valeurs de différence (M) d'une valeur de mesure (M) qui dépend du volume systolique, lorsque la valeur des variations ($\Delta f$) de la fréquence augmente, de manière à détecter une variation maximale ($\Delta M_{max}$) de la valeur de mesure pour $\Delta f_o = \Delta 1/t_{so}$.

8. Dispositif suivant la revendication 6, caractérisé par le fait que l'on compare la différence entre deux valeurs maximales de variation de la valeur de mesure pour deux fréquences de base différentes (f1) et (f2), mais pour une même charge, d'une manière rapportée à la valeur ($\Delta M_{max}$) pour la fréquence (f1) au quotient ($\Delta f/f + f$), et qu'on utilise la valeur de comparaison pour réaliser une optimisation hémodynamique de la régulation de la fréquence.

9. Dispositif suivant l'une des revendications 1 à 4, caractérisé par le fait que l'on compare des quotients de brèves variations de paramètres de mesure, qui dépendent du volume systolique, pour une même fréquence de base, à des valeurs de consigne mémorisées du quotient pour cette fréquence et qu'on analyse de cette manière l'efficacité du muscle cardiaque pour réaliser la détection d'une tachycardie.

10. Dispositif suivant la revendication 1 ou 2, caractérisé par le fait qu'on utilise les différences de variations de brève durée de paramètres de mesure qui dépendent de la fréquence et de la charge, pendant une charge constante, à des variations de durée moyenne de ces paramètres, après une variation de la charge et qu'on les utilise pour séparer la dépendance de ces paramètres vis-à-vis de la fréquence et leur dépendance vis-à-vis de la charge.

FIG 1

EKG

QRS  T  n  n+1

V

$SV_{max}$

$SV_n$  $SV_{n+1}$

0

$t_{s0}$  $\Delta t_{s0}$  $\Delta t_{s0}$  $\Delta t_s$

$t_s$  $t_s + \Delta t_s$

11

# FIG 2

# FIG 3

# FIG 4

# FIG 5